# EUROPEAN PATENT SPECIFICATION

(11) **EP 3 012 241 B1**
(45) Date of publication and mention of the grant of the patent: **31.10.2018**
(21) Application number: 14813057.8
(22) Date of filing: 16.06.2014
(51) Int. Cl.: C07C 19/08, C07C 17/383, C07C 17/389, H01L 21/3065, H01L 21/311

(54) **PLASMA ETCHING METHOD WITH HIGH-PURITY 1-FLUOROBUTANE**
PLASMAÄTZVERFAHREN DURCH VERWENDUNG VON HOCHREINEM 1-FLUOROBUTAN
PROCÉDÉ DE GRAVURE AU PLASMA EN UTILISANT 1-FLUOROBUTANE DE PURETÉ ÉLEVÉE

(30) Priority: 17.06.2013 JP 2013126240
(43) Date of publication of application: 27.04.2016
(73) Proprietor: Zeon Corporation, Tokyo 100-8246 (JP)
(72) Inventor: SUGIMOTO, Tatsuya, Tokyo 100-8246 (JP)
(74) Representative: Beckmann, Claus
(86) International application number: PCT/JP2014/065847
(87) International publication number: WO 2014/203842

(56) References cited:
- WO-A1-2009/123038
- JP-A- 2013 006 786
- JP-A- 2014 024 785
- US-A1- 2011 068 086
- US-A1- 2013 105 916
- HOOPER D L ET AL: "The NMR spectra of the n-alkyl fluorides", JOURNAL OF MOLECULAR SPECTROSCOPY, ACADEMIC PRESS, US, vol. 24, no. 1-4, 1 January 1967 (1967-01-01), pages 277-283, XP023963776, ISSN: 0022-2852, DOI: 10.1016/0022-2852(67)90091-4 [retrieved on 1967-01-01]
- ROBERT DOMINIQUE U ET AL: "Phenyltetrafluorophosphorane as a selective fluorination agent for alcohols", TETRAHEDRON LETTERS, vol. 13, no. 9, 1972, pages 847-849, XP029122144, ISSN: 0040-4039, DOI: 10.1016/S0040-4039(01)84454-3
- KAGAKU DAIJITEN vol. 7, 30 October 1961, page 878, XP008182590

## Description

### TECHNICAL FIELD

The present invention relates to a dry etching method using 1-fluorobutane as defined in the claims. High-purity 1-fluorobutane is suitable as a plasma etching gas and a plasma reaction gas (e.g., CVD gas) that is used when producing a semiconductor device utilizing a plasma reaction.

### BACKGROUND ART

Semiconductor production technology that achieves further miniaturization has been developed, and a line width of 20 nm or 10 nm has been used for a leading-edge process. The degree of difficulty in processing has increased along with an increase in the degree of miniaturization, and various techniques are currently under development using various approaches in terms of the materials, devices, and processing methods.

In view of the above situation, the applicant of the present application developed a plasma etching gas that includes a saturated fluorohydrocarbon represented by the formula (1): CₓH_{y}F_{z} (wherein x is 3, 4, or 5, and y and z are independently a positive integer, provided that y>z) that can deal with a leading-edge dry etching process, and reported that a saturated fluorohydrocarbon having a small number of fluorine atoms exhibits a performance better than that of monofluoromethane that is used to etch a silicon nitride film (see Patent Document 1).

The following methods are known as a method for producing 1-fluorobutane (i.e., a saturated fluorohydrocarbon represented by the formula (1)), for example. Patent Document 2 discloses converting 1-butanol into 1-butylmethanesulfonate, and reacting 1-butylmethanesulfonate with potassium fluoride in propylene glycol to obtain 1-fluorobutane. Non-Patent Document 1 discloses converting 1-butanol into trimethylsiloxybutane, and bringing trimethylsiloxybutane into contact with phenyltetrafluorophosphorane (i.e., fluorinating agent) to obtain 1-fluorobutane in a yield of 35%. Non-Patent Document 2 discloses heating a mixture including tetrabutylammonium bromide and cesium fluoride to prepare tetrabutylammonium fluoride within the system, and adding 1-bromobutane to the system to obtain 1-fluorobutane in a yield of 77%.

### RELATED-ART DOCUMENT

### PATENT DOCUMENT

Patent Document 1: WO2009/123038 (US2011/068086)
Patent Document 2: JP-A-2013-6786

### NON-PATENT DOCUMENT

Non-Patent Document 1: Tetrahedron, Vol. 29, 1877 (1973)
Non-Patent Document 2: Journal of Fluorine Chemistry, Vol. 73, 185 (1995)

### SUMMARY OF THE INVENTION

### TECHNICAL PROBLEM

The inventor of the invention used 1-fluorobutane obtained using the above methods as a gas for selectively dry-etching a silicon nitride film stacked on silicon or a silicon oxide film. However, a large amount of hydrocarbon-based deposits were produced, and etching stopped.

An object of the invention is to solve the above problem, and provide a dry etching method that uses 1-fluorobutane as an etching gas that can selectively dry-etch a silicon nitride film or the like that is stacked on silicon or a silicon oxide film.

### SOLUTION TO PROBLEM

The inventor conducted extensive studies in order to solve the above problem. As a result, the inventor found that the above problem occurs when the butene content in 1-fluorobutane is equal to or higher than a specific value. This finding has led to the completion of the invention.

One aspect of the invention provides a dry etching method comprising using 1-fluorobutane having a purity of 99.9% by volume or more and a total butene content of 1,000 ppm by volume or less, a nitrogen content of 100 ppm by volume or less and an oxygen content of 50 ppm by volume or less. The 1-fluorobutane preferably has a water content of 50 ppm by volume or less.

### DESCRIPTION OF EMBODIMENTS

1-Fluorobutane used in the method according to the the invention has a purity of 99.9% by volume or more and a butene content of 1,000 ppm by volume or less, a nitrogen content of 100 ppm by volume or less and an oxygen content of 50 ppm by volume or less.

Note that the term "butene" used herein is a generic name for 1-butene, 2-butene ((E)-2-butene and (Z)-2-butene), and isobutene. One or more types of butenes are present in the 1-fluorobutane as impurities.

The purity of the 1-fluorobutane and the butene content in the 1-fluorobutane refer to values calculated from the peak area in a chart obtained by subjecting the 1-fluorobutane to gas chromatography using a flame ionization detector (FID). The butene may be identified by gas chromatography-mass spectrometry. The nitrogen content and the oxygen content in the 1-fluorobutane refer to values determined by gas chromatography using a thermal conductivity detector (TCD). The water content in the 1-fluorobutane refers to a value determined by FT-IR.

The 1-fluorobutane according to one embodiment of the invention may be obtained by purifying crude 1-fluorobutane (produced using a known method) by means of distillation to remove the butene (impurities).

The 1-fluorobutane may be produced using an arbitrary method. For example, the crude 1-fluorobutane may be produced using (i) a method that fluorinates 1-butanol using a fluorinating agent, (ii) a method that treats 1-bromobutane or a butyl alkylsulfonate with an alkali metal fluoride (e.g., potassium fluoride or cesium fluoride), or the like.

The crude 1-fluorobutane produced using the above method is purified by means of distillation (rectification) or the like to remove organic impurities including the butene. The butane content can be reduced to 1,000 ppm by volume or less, and preferably 500 ppm by volume or less, by rectifying the crude 1-fluorobutane.

A rectifying column is used when purifying the crude 1-fluorobutane by means of distillation to remove organic impurities. A rectifying column having an appropriate number of theoretical plates is used to efficiently separate 1-fluorobutane (boiling point: 32°C) from the butene (1-butene (boiling point: -6.3°C), (E)-2-butene (boiling point: 0.9°C), and (Z)-2-butene (boiling point: 3.7°C)). The number of theoretical plates is normally about 10 to about 50, and preferably 20 to 50. Since the boiling point of the butene is equal to or less than room temperature, the efficiency of separation from the target 1-fluorobutane may (apparently) deteriorate due to a vaporization phenomenon within a fraction extraction line of the rectifying column. Therefore, it is preferable to sufficiently cool the fraction extraction line and a first fraction storage container (to a temperature equal to or lower than the boiling point of the butene).

The rectification pressure (gauge pressure) is normally set to a value between normal pressure and 1013 kPa (10 atmospheres) and preferably set to a value between normal pressure and about 506.5 kPa (5 atmospheres). The ratio of the reflux rate to the distillate rate (hereinafter may be referred to as "reflux ratio") is preferably set to 30:1 or more in order to efficiently separate the butene that easily gasifies. If the reflux ratio is too low, it may be difficult to efficiently separate the butene, and sufficiently increase the purity of 1-fluorobutane. Moreover, the amount of the first fraction may increase, and the total amount of 1-fluorobutane (collected as a product) may decrease. If the reflux ratio is too high, collection (per extraction) may take time, and the rectification time may increase. As a result, productivity may deteriorate.

A batch-wise purification method or a continuous purification method may be used. A batch-wise purification method is preferably used when the production volume is small. When the production volume is large, a continuous purification method that utilizes several rectifying columns is preferably used. An extractive distillation operation that utilizes an extraction solvent may be performed in combination with rectification.

When the reaction conversion ratio is low, and it is necessary to collect the raw material, for example, a stepwise distillation operation (that separates the raw material compound by the first distillation operation, and separates the butene (impurities) by the second distillation operation, for example) may be performed depending on the reaction used to produce 1-fluorobutane. In this case, it is preferable to set the reflux ratio to 40:1 or more.

The nitrogen content in the 1-fluorobutane used in the method according to the invention is 100 ppm by volume or less, and more preferably 80 ppm by volume or less. The oxygen content in the 1-fluorobutane used in the method according to the invention is 50 ppm by volume or less, and more preferably 30 ppm by volume or less.

Nitrogen and oxygen included in the 1-fluorobutane may be removed by removing the butene by means of rectification using a Group 0 gas (inert gas), or subjecting the 1-fluorobutane to simple distillation, and extracting a fraction, for example. When using the latter method, the nitrogen content and the oxygen content in the 1-fluorobutane that remains in the still can be reduced by subjecting the 1-fluorobutane to simple distillation, and removing nitrogen and oxygen together with the 1-fluorobutane. The amount of the 1-fluorobutane that is extracted is preferably 20 to 50 wt%, and more preferably 30 to 40 wt%, based on the 1-fluorobutane that is put into the still. The extracted 1-fluorobutane may be stored, and added to the next batch (i.e., recycled).

The 1-fluorobutane according to one embodiment of the invention preferably has a water content of 50 ppm by volume or less, and more preferably 20 ppm by volume or less.

Water included in the 1-fluorobutane may be removed using a normal method such as a method that brings the 1-fluorobutane into contact with an adsorbent.

A molecular sieve, alumina, or the like may be used as the adsorbent. It is preferable to use a 3A molecular sieve when drying a monofluorohydrocarbon (e.g., 1-fluorobutane) (see JP-A-2014-24785 (Japanese Patent Application No. 2012-165797)). When a molecular sieve having a large pore size (e.g., 4A or 5A molecular sieve) is used, the 1-fluorobutane molecules may enter the pores, and the effect of reducing the water content may decrease. When an alkaline molecular sieve is used, the 1-fluorobutane may undergo a dehydrofluorination reaction. Therefore, it is necessary to carefully select the molecular sieve. When using alumina, it is preferable to use activated alumina that has low crystallinity and is produced by subjecting alumina hydrate to thermal dehydration.

It is preferable to activate the adsorbent (e.g., molecular sieve or alumina) by means of calcination or the like before bringing the 1-fluorobutane into contact with the adsorbent, since the adsorbent can adsorb a larger amount of water.

The water content in the 1-fluorobutane can be reduced to 50 ppm by volume or less by bringing the 1-fluorobutane into contact with the adsorbent. If the water content in the 1-fluorobutane is high, water may adhere to (remain on) the processing target surface of a substrate after etching, and delamination of a laminate film may occur when forming a copper wire or the like, or the embedded wire may be corroded. Therefore, it is preferable to reduce the water content in the 1-fluorobutane as much as possible.

As described above, it is possible to obtain high-purity 1-fluorobutane that is suitable as a plasma reaction gas by performing a step (I) that rectifies the crude 1-fluorobutane included in the crude reaction product to have a purity of 99.9% by volume or more and a butene content of 1,000 ppm by volume or less, a step (II) that removes water from the resulting 1-fluorobutane by bringing the resulting 1-fluorobutane into contact with the adsorbent, and a step (III) that subjects the resulting 1-fluorobutane to simple distillation to reduce the nitrogen content and the oxygen content in the 1-fluorobutane to 100 ppm by volume or less and 50 ppm by volume or less, respectively. It is preferable to perform the steps (I) to (III), from the viewpoint of efficiently obtaining the 1-fluorobutane according to one embodiment of the invention.

It is possible to improve processing stability during dry etching by thus reducing the impurity content in the 1-fluorobutane to be equal to or lower than a specific value. The 1-fluorobutane according to one embodiment of the invention can also be applied to an inorganic nitride film (e.g., silicon oxynitride, titanium nitride, and aluminum nitride) in addition to a silicon nitride film.

A plasma etching method according to one embodiment of the invention uses the 1-fluorobutane disclosed as an etching gas. The etching gas used for the plasma etching method according to one embodiment of the invention may include only the 1-fluorobutane disclosed, or may further include oxygen gas and/or nitrogen gas. It may be possible to significantly increase the selectivity ratio while preventing an etching stop phenomenon that is considered to occur due to accumulation (deposition) of reaction products at the bottom of a hole by utilizing oxygen gas and/or nitrogen gas in addition to the 1-fluorobutane.

The volume ratio of oxygen gas, nitrogen gas, or oxygen gas and nitrogen gas in total to the 1-fluorobutane is preferably 0.1:1 to 50:1, and more preferably 0.5:1 to 30:1.

At least one Group 18 gas selected from the group consisting of helium, argon, neon, krypton, and xenon may further be used as the process gas. It may be possible to increase the etching rate of an inorganic nitride film while maintaining the selectivity ratio by additionally utilizing the Group 18 gas.

The volume ratio of the Group 18 gas to the 1-fluorobutane is preferably 0:1 to 100:1, and more preferably 0:1 to 20:1.

The process gas is fed (introduced) at a rate proportional to the amount of each component. For example, the 1-fluorobutane gas is fed at 8×10⁻³ to 5×10⁻² Pa·m³/sec, oxygen gas is fed at 8×10⁻² to 5×10⁻¹ Pa·m³/sec, and the Group 18 gas is fed at 8×10⁻² to 5×10⁻¹ Pa·m³/sec.

The pressure inside the chamber into which the process gas has been introduced is normally 0.0013 to 1,300 Pa, and preferably 0.13 to 13 Pa.

When a high-frequency electric field is applied to the 1-fluorobutane gas (reactive plasma gas) included in the chamber using a plasma generator, a glow discharge occurs so that a plasma is generated.

Examples of the plasma generator include a helicon wave-type plasma generator, a high-frequency induction-type plasma generator, a parallel plate-type plasma generator, a magnetron-type plasma generator, a microwave-type plasma generator, and the like. It is preferable to use a helicon wave-type plasma generator, a high-frequency induction-type plasma generator, or a microwave-type plasma generator since a high-density plasma can be easily generated.

The plasma density is not particularly limited. It is preferable to etch the processing target in a high-density plasma atmosphere having a plasma density of 10¹¹ ions/cm³ or more, and more preferably 10¹² to 10¹³ ions/cm³, in order to more easily achieve the advantageous effects of the invention.

The temperature of the etching target substrate that is reached during etching is not particularly limited, but is preferably 0 to 300°C, more preferably 0 to 100°C, and still more preferably 20 to 80°C. The temperature of the substrate may or may not be controlled by means of cooling or the like.

The etching time is normally 5 to 10 minutes. Since the process gas used in connection with one embodiment of the invention enables high-speed etching, the etching time may be set to 2 to 5 minutes to improve productivity.

As described above, the plasma etching method according to one embodiment of the invention (that generates a plasma in a chamber using an etching gas, and etches a specific part of the processing target disposed within the chamber) utilizes the process gas (etching gas) that includes the 1-fluorobutane. The plasma etching method according to one embodiment of the invention is preferably used to selectively plasma-etch an inorganic nitride film, and more preferably used to selectively plasma-etch a silicon nitride film. For example, the plasma etching method according to one embodiment of the invention is used to selectively plasma-etch a silicon nitride film relative to a silicon oxide film.

The selectivity ratio of a silicon nitride film to a silicon oxide film can be increased to 10 or more (20 or more in many cases) by etching a silicon nitride film under the above etching conditions. Specifically, a significantly high selectivity ratio can be obtained as compared with a known method while preventing an etching stop phenomenon due to deposits. This makes it possible to prevent a situation in which a silicon oxide film (SiO₂ film) breaks while etching a silicon nitride film, even if the thickness of a silicon oxide film included in a device is reduced. Therefore, it is possible to reliably etch only a silicon nitride film, and produce a device that exhibits excellent electrical properties.

The plasma etching method according to one embodiment of the invention may be applied (a) when forming a mask pattern so that a given area of an ONO film (silicon oxide film-silicon nitride film-silicon oxide film) is exposed, etching the ONO film via the mask pattern to remove at least the upper silicon oxide film, and selectively etching the exposed silicon nitride film, or (b) when forming a thin silicon nitride film (e.g., 10 to 20 nm) on the side wall (inner wall) of a contact hole in order to protect an interlayer dielectric (oxide film) against damage, and etching away part of the silicon nitride film situated at the bottom of the contact hole, for example.

### EXAMPLES

The invention is further described below by way of examples. Note that the scope of the invention is not limited to the following examples.

The following analysis conditions were used in connection with the examples.
(1) Gas chromatography analysis (GC analysis)
   Device: HP-6890 manufactured by Agilent Technologies
   Column: Inert Cap-1 manufactured by GL Sciences Inc. (length: 60 m, inner diameter 0.25 mm, thickness: 1.5 µm)
   Column temperature: held at 40°C for 10 minutes, heated to 240°C at 20°C/min, and held at 240°C for 10 minutes
   Injection temperature: 200°C
   Carrier gas: nitrogen
   Split ratio: 100/1
   Detector: FID
(2) Analysis of impurities (gas chromatography-mass spectrometry)
   GC device: HP-6890 manufactured by Agilent Technologies
   Column: Inert Cap-1 manufactured by GL Sciences Inc. (length: 60 m, inner diameter 0.25 mm, thickness: 1.5 µm)
   Column temperature: held at 40°C for 10 minutes, heated to 240°C at 20°C/min, and held at 240°C for 10 minutes
   MS device: 5973 NETWORK manufactured by Agilent Technologies
   Detector: EI detector (accelerating voltage: 70 eV)
(3) ¹H-NMR analysis and ¹⁹F-NMR analysis
   Device: JNM-ECA-400 manufactured by JEOL Ltd. (400 MHz)
(4) Measurement of nitrogen content and oxygen content (gas chromatography)
   GC device: HP-7890 manufactured by Agilent Technologies
   Column: HP-5 manufactured by Agilent Technologies (length: 30 m, inner diameter 0.32 mm, thickness: 0.25 µm)
   Column temperature: held at 40°C for 5 minutes, heated to 65°C at 5°C/min, and held at 65°C for 1 minute
   Gas sampler: 50°C
   Carrier gas: helium
   Detector: TCD+pulse discharge detector
(5) Measurement of water content (FT-IR)
   IG-1000 manufactured by Otsuka Electronics Co., Ltd.
   Cell length: 10 m

### Production Example 1

A 2 L glass reactor equipped with a stirrer, a dropping funnel, and a Dimroth condenser was charged with 1-butanol (74 g), methanesulfonyl chloride (126 g), and dry diisopropyl ether (500 mL). The mixture was subjected to a nitrogen atmosphere. The reactor was cooled with ice water, and triethylamine (121 g) was added dropwise to the mixture from the dropping funnel over about 2 hours. After the dropwise addition, the mixture was stirred at 0°C for 30 minutes, and then stirred at 25°C for 6 hours.

500 mL of ice water was added to the reaction mixture to dissolve triethylamine hydrochloride produced to separate the reaction mixture into two layers. The upper organic layer was sequentially washed with 5% hydrochloric acid, saturated sodium bicarbonate water, and a saturated sodium chloride solution, and dried over anhydrous magnesium sulfate, and magnesium sulfate was filtered off. Diisopropyl ether was evaporated from the filtrate using a rotary evaporator, followed by pumping up using a vacuum pump to obtain 119 g of crude methanesulfonyloxybutane.

### Production Example 2

A 1 L glass reactor equipped with a stirrer, a dropping funnel, a fraction receiver, and a Dimroth condenser was charged with 116 g of spray-dried potassium fluoride (manufactured by Aldrich) and 400 mL of propylene glycol. The mixture was subjected to a nitrogen atmosphere. The reactor was immersed in an oil bath, and heated to 90°C, and 120 g of the crude methanesulfonyloxybutane obtained as described above (see Production Example 1) was added to the mixture from the dropping funnel over about 3.5 hours. After the dropwise addition, the mixture was stirred at 90°C for 2 hours, and the resulting low-boiling-point product was collected into the fraction receiver immersed in a dry ice-ethanol bath. After lowering the temperature of the oil bath to 80°C, two glass traps immersed in a dry ice-ethanol bath were connected to the reactor in series. A pressure controller and a vacuum pump were connected to the outlet of the glass traps. The vacuum pump was operated, and the pressure inside the system was lowered stepwise to 50 to 45 kPa, 35 to 30 kPa, and 30 to 25 kPa using the pressure controller to collect volatile components into the glass traps. The contents of the fraction receiver and the two glass traps were combined, and analyzed by gas chromatography. It was found that the mixture included 1-butene (3.47% by area), (E)-2-butene (0.31% by area), (Z)-2-butene (0.29% by area), 1-fluorobutane (87.82% by area), diisopropyl ether (3.53% by area), and a high-boiling-point component (4.58% by area).

### Example 1 (not part of the invention)

A still was charged with 598 g of crude 1-fluorobutane obtained as described above (see Production Examples 1 and 2), and a distillation operation was performed using a KS rectifying column (manufactured by Toka Seiki Co., Ltd., column length: 60 cm, packing material: Heli Pack No. 1). A refrigerant (-10°C) was circulated through a condenser, and total reflux was effected for about 1 hour. The still was heated at 45 to 70°C while observing the temperature of the top of the column and the amount of the crude 1 -fluorobutane remaining in the still. A fraction was then extracted at a reflux ratio of 45:1. 508 g of 1-fluorobutane (99.93% by area (volume)) was thus obtained. The 1-fluorobutane included 1-butene (612 ppm by area (volume)), (E)-2-butene (33 ppm by area (volume)), and (Z)-2-butene (55 ppm by area (volume)) as impurities.

The spectral data of the resulting 1-fluorobutane are shown below.
¹H-NMR (CDCl₃, TMS) δ (ppm): 0.95 (t, 3H), 1.43 (m, 2H), 1.70 (m, 2H), 4.45 (m, 2H) ¹⁹F-NMR (CDCl_{3,} CFCl₃) δ (ppm): -219 (m, F)

### Example 2 (not part of the invention)

A 1.2 L SUS316 container (electropolished on the inner surface) charged with 100 g of a 3A molecular sieve (manufactured by Wako Pure Chemical Industries, Ltd.) was charged with 463 g of the 1-fluorobutane obtained in Example 1 (through purification by means of distillation), and the mixture was allowed to stand at room temperature (25°C) for 22 hours to remove water.

A simple distillation apparatus (in which a short column, a condenser, and a receiver were provided over a SUS316 still (capacity: 1 L)) was provided, and cooling water (-10°C) was circulated through the condenser. 419 g of the 1-fluorobutane from which water had been removed was put into the still, and the still was heated to 60°C.

The nitrogen content (concentration) and the oxygen content (concentration) in the 1-fluorobutane determined by gas chromatography were 634 ppm by volume and 150 ppm by volume, respectively.

The simple distillation operation was terminated when about 30 mass% of the 1-fluorobutane had been extracted into the receiver, and the still was cooled to room temperature. A 1 L manganese steel cylinder (inner surface roughness: 1S) equipped with a diaphragm-type valve was charged with 290 g of the 1-fluorobutane included in the still. The purity of the 1-fluorobutane was 99.9% by volume or more. The 1-fluorobutane included 1-butene (541 ppm by area (volume)), (E)-2-butene (30 ppm by area (volume)), and (Z)-2-butene (48 ppm by area (volume)). The nitrogen content, the oxygen content, and the water content in the 1-fluorobutane were 58 ppm by volume, 12 ppm by volume, and 22 ppm by volume, respectively.

### Example 3 (not part of the invention)

A still was charged with 389 g of the crude 1-fluorobutane obtained as described above (see Production Example 1), and a distillation operation was performed using a KS rectifying column (manufactured by Toka Seiki Co., Ltd., column length: 60 cm, packing material: Heli Pack No. 1). A refrigerant (-10°C) was circulated through a condenser, and total reflux was effected for about 1 hour. The still was heated from 45°C to 70°C while observing the temperature of the top of the column and the amount of the crude 1-fluorobutane remaining in the still. A fraction was then extracted at a reflux ratio of 30:1. 329 g of 1-fluorobutane (99.91% by area (volume)) was thus obtained. The 1-fluorobutane included 1-butene (788 ppm by area (volume)), (E)-2-butene (56 ppm by area (volume)), and (Z)-2-butene (72 ppm by area (volume)).

### Example 4 (not part of the invention)

A 1.2 L stainless steel container charged with 60 g of alumina ("N612N" manufactured by JGC Catalysts and Chemicals Ltd.) was charged with 329 g of the 1-fluorobutane obtained in Example 3, and the mixture was allowed to stand at room temperature (25°C) for 20 hours.

The stainless steel container was connected to a 1 L manganese steel cylinder through a stainless steel tube, and the cylinder was charged with the 1-fluorobutane under reduced pressure through a metal filter having a pore size of 0.2 µm. The cylinder was cooled with ice water, and about 30 g of the 1-fluorobutane was extracted under a pressure of 5 to 10 kPa while reducing the pressure using a vacuum pump via a pressure controller. After returning the temperature inside the stainless steel container to room temperature (25°C), the container was allowed to stand for a while. The purity of the 1-fluorobutane was 99.9% by volume or more. The 1-fluorobutane included 1-butene (716 ppm by area (volume)), (E)-2-butene (51 ppm by area (volume)), and (Z)-2-butene (65 ppm by area (volume)). The nitrogen content, the oxygen content, and the water content in the 1-fluorobutane were 45 ppm by volume, 14 ppm by volume, and 40 ppm by volume, respectively.

### Reference Example 1

A still was charged with 604 g of the crude 1-fluorobutane obtained as described above (see Production Examples 1 and 2), and a distillation operation was performed using a KS rectifying column (manufactured by Toka Seiki Co., Ltd., column length: 60 cm, packing material: Heli Pack No. 1). A refrigerant (-10°C) was circulated through a condenser, and total reflux was effected for about 1 hour. The still was heated at 45 to 70°C while observing the temperature of the top of the column and the amount of the crude 1-fluorobutane remaining in the still. A fraction was then extracted at a reflux ratio of 10:1. 282 g of 1-fluorobutane (99.85% by area) was thus obtained. The 1-fluorobutane included 1-butene (1,281 ppm by area (volume)), (E)-2-butene (107 ppm by area (volume)), and (Z)-2-butene (112 ppm by area (volume)) as impurities. A cylinder was charged with 232 g of the 1-fluorobutane in the same manner as in Example 4. The nitrogen content, the oxygen content, and the water content in the 1-fluorobutane were determined, and found to be 43 ppm by volume, 10 ppm by volume, and 29 ppm by volume, respectively.

### Dry etching evaluation

A wafer on which a silicon nitride film was formed, and a wafer on which a silicon oxide film was formed, were respectively dry-etched using the 1-fluorobutane obtained in Example 2, the 1-fluorobutane obtained in Example 4, or the 1-fluorobutane obtained in Reference Example 1 as an etching gas.

The etching rate of the silicon nitride film and the etching rate of the silicon oxide film were measured, and the selectivity ratio (SiN film/SiO₂ film) was calculated from the ratio of the etching rate of the silicon nitride film to the etching rate of the silicon oxide film.

Each wafer was dry-etched as described below.

Specifically, the wafer on which a silicon nitride film was formed, or the wafer on which a silicon oxide film was formed, was placed in an etching chamber of a parallel plate-type plasma etching device. After evacuating the system, the silicon nitride film or the silicon oxide film was etched under the following etching conditions using the 1-fluorobutane obtained in Example 2, the 1-fluorobutane obtained in Example 4, or the 1-fluorobutane obtained in Reference Example 1. The results are shown in Table 1.

### Etching conditions

Mixed gas pressure: 6.7 Pa
Power supplied to upper electrode from high-frequency power supply: 200 W
Power supplied to lower electrode from high-frequency power supply: 100 W
Interval between upper electrode and lower electrode: 50 mm
Electrode temperature: 20°C
Gas flow rate
O₂ gas: 60 sccm
1-Fluorobutane: 40 sccm
Etching time: 180 sec

**TABLE 1**

| 1-fluorobutane | Etching rate (nm/min) | | Selectivity (SiN film/SiO₂ film) |
|---|---|---|---|
| | SiN film | SiO₂ film | |
| Example 2 | 28 | Not etched | Infinity |
| Example 4 | 25 | Not etched | Infinity |
| Reference Example 1 | Deposition occurred (etching stopped) | Not etched | - |

## Claims

1. A dry etching method comprising using 1-fluorobutane as an etching gas, the 1-fluorobutane having a purity of 99.9% by volume or more, a total butene content of 1,000 ppm by volume or less, a nitrogen content of 100 ppm by volume or less and an oxygen content of 50 ppm by volume or less.

2. The dry etching method according to claim 1, wherein the 1-fluorobutane has a water content of 50 ppm by volume or less.

## Patentansprüche

1. Trockenätzverfahren, das eine Verwendung von 1-Fluorbutan als Ätzgas umfasst, wobei das 1-Fluorbutan eine Reinheit von 99,9 Volumen-% oder mehr, einen Gesamtbutengehalt von 1.000 Volumen-ppm oder weniger, einen Stickstoffgehalt von 100 Volumen-ppm oder weniger und einen Sauerstoffgehalt von 50 Volumen-ppm oder weniger hat.

2. Trockenätzverfahren gemäß Anspruch 1, wobei das 1-Fluorbutan einen Wassergehalt von 50 Volumen-ppm oder weniger hat.

## Revendications

1. Procédé de gravure à sec comprenant l'utilisation du 1-fluorobutane comme gaz de gravure, ledit 1-fluorobutane ayant une pureté de 99,9% en volume ou plus, une teneur totale en butène de 1000 ppm en volume ou moins, une teneur en azote de 100 ppm en volume ou moins et une teneur en oxygène de 50 ppm en volume ou moins.

2. Procédé de gravure à sec selon la revendication 1, dans lequel ledit 1-fluorobutane a une teneur en eau de 50 ppm en volume ou moins.
